# EUROPEAN PATENT SPECIFICATION

(11) **EP 2 826 476 B1**
(45) Date of publication and mention of the grant of the patent: **03.03.2021**
(21) Application number: 13176623.0
(22) Date of filing: 16.07.2013
(51) Int. Cl.: A61K 31/194, A61K 31/7004, A61K 33/10, A61K 33/14, A61P 7/08, A61M 1/16, A61M 1/36

(54) **Calcium-free dialysis fluid**
Calciumfreie Dialyseflüssigkeit
Fluide de dialyse exempt de calcium

(43) Date of publication of application: 21.01.2015
(73) Proprietor: G.M.T. de Jong B.V., 3311 KV Dordrecht (NL); van der Meulen, Jan, 3328 AM Dordrecht (NL); M.R. Korte B.V., 4177 CC Herwijnen (NL)
(72) Inventor: de Jong, Gijsbertus Maria Theodorus, 3311 KV Dordrecht (NL); van der Meulen, Jan, 3328 AM Dordrecht (NL); Korte, Mario Richard, 4177 CC Herwijnen (NL)
(74) Representative: V.O.

(56) References cited:
- DE-A1- 4 114 908
- DE-A1- 19 654 746
- US-A- 4 500 309
- GABUTTI LUCA ET AL: "Citrate- vs. acetate-based dialysate in bicarbonate haemodialysis: consequences on haemodynamics, coagulation, acid-base status, and electrolytes", BMC NEPHROLOGY, BIOMED CENTRAL, LONDON, GB, vol. 10, no. 1, 5 March 2009 (2009-03-05), page 7, XP021048858, ISSN: 1471-2369, DOI: 10.1186/1471-2369-10-7
- Annie Tu ET AL: "Heparin-Free Hemodialysis with Citrate-Containing Dialysate in Intensive Care Patients", Dialysis and Transplantation, vol. 29, no. 10, 31 October 2000 (2000-10-31), pages 620-624, XP055516431,
- Y.-L. Cheng ET AL: "Anticoagulation during haemodialysis using a citrate-enriched dialysate: a feasibility study", NEPHROLOGY DIALYSIS TRANSPLANTATION., vol. 26, no. 2, 8 July 2010 (2010-07-08), pages 641-646, XP055516433, GB ISSN: 0931-0509, DOI: 10.1093/ndt/gfq396
- "Citrasate Concentrate", USERS MANUAL, RENALFARMA, LT , 1 January 2010 (2010-01-01), pages 1-13, XP009508786, Retrieved from the Internet: URL:www.renalfarma.lt/download.php/fileid/ 75

## Description

The invention relates to a dialysis fluid, to a dialysis fluid for use in in a hemodialysis therapy or hemodiafiltration therapy, to a concentrate for preparing a dialysis fluid and to a method for extracorporeally dialyzing blood by either hemodialysis or hemodiafiltration.

Treatment of end-stage renal diseases usually involves hemodialysis (HD), hemodiafiltration (HDF), or renal transplantation. In HD and HDF patient's blood passes through a dialyzer containing a semi-permeable membrane. The semi-permeable membranes can be arranged in various configurations, such as, for example, bundles of hollow fibres. This membrane keeps the blood separated from the cleaning solution, *i*.*e*. the dialysis fluid. Various dialysis fluids are generally known in the art. They typically contain dissolved salts (ions). Also they may contain glucose. Uremic toxins and ions in the blood diffuse out of the blood through the semi-permeable membrane into the dialysis fluid. The dialysis fluid with the uremic toxins leaving the dialyzer is called dialysate. The concentration of ions and glucose (if present) in the dialysis fluid prevent diffusion of these elements from the blood to such an extent that the blood levels may become too low. This prevents e.g. potassium concentration becoming dangerously low. Dissolved substances that are present in the dialysis fluid in a higher concentration than in plasma, will diffuse into the blood, permitted that the semi-permeable membrane is permeable to them. The amount that will diffuse is dependent on the concentration gradient across the membrane, the available surface of the membrane and the flow of blood and dialysis fluid. In addition to diffusion, in HDF a large volume of plasma is filtered, whereby larger molecules are cleared from the plasma with as a consequence plasma fluid loss. In this modality, the plasma fluid loss is replaced by the dialysis fluid. This fluid is infused directly into the bloodstream through the dialysis machine.

When a patient's blood passes through the hollow fibres in the dialyzer during HD or HDF, it is extracorporeal and exposed to xenobiotic surfaces. Such surfaces exhibit a variable degree of thrombogenicity and may initiate clotting of patient's blood, especially in conjunction with exposure of blood to air in the air trap. The resulting thrombus formation may cause loss of blood and occlusion of the extracorporeal circuit necessitating stopping the HD or HDF.

In order to prevent blood coagulation in the extracorporeal circuit during HD and HDF, usually an anticoagulant is added to the blood before it enters the dialyzer. Most commonly, unfractionated or fractionated heparin is used for this purpose. A drawback of heparin is that its use leads to systemic anticoagulation (*i.e.* anticoagulation of the patient), giving rise to the risk of serious bleeding complications, particularly in critically ill patients. Minor potential undesirable side effects of heparin are pruritus, allergy, osteoporosis, hyperlipidemia, and thrombocytopenia. Heparin is therefore not recommended for patients at risk of bleeding due to concomitant use of other anticoagulants (e.g. vitamin K antagonists, thrombin inhibitors, or prostaglandin inhibitors), gastrointestinal lesions (gastritis, peptic ulcer, and angiodysplasia), recent surgery, pericarditis or an increased risk for cerebral microbleeds.

Another method of preventing blood coagulation is to administer citrate to the patient's blood entering the dialysis machine. Citrate forms complexes with ionized calcium (Ca²⁺ that has not formed a complex with another compound, in particular a protein; herein after shortly referred to as 'iCa'), which is factor IV in the coagulation cascade and thereby citrate inhibits clotting. However, citrate forms also complexes with ionized magnesium, another divalent cation (Mg²⁺ that has not formed a complex with another compound, in particular a protein; herein after shortly referred to as ' iMg'). Thus iMg counteracts the anticoagulative effect of citrate because part of the citrate is bound to magnesium and not available for binding factor IV. If a dialysis fluid is used that lacks either iCa, or lacks iCa as well as iMg, the anticoagulative effect remains throughout the complete passage of the blood through the dialyzer.

Systemic anticoagulation due to the presence of citrate is effectively avoided by using a calcium containing substitution fluid, which substitution fluid may also contain magnesium, which is added to the blood after the dialyzer before it is returning into the patient. In this way citrate's anticoagulative effect is restricted to the extracorporeal system, *i*.*e*. regional anticoagulation.

Another advantage of citrate over heparin is that it improves biocompatibility. Citrate has been shown to reduce complement activation, degranulation of granulocytes and platelets and the release of IL-1b.

One way of using citrate as anticoagulant in HD or HDF involves citrate infusion before the dialyzer, calcium infusion after the dialyzer and use of a calcium-free, yet magnesium containing and acetate containing dialysis fluid. This method is generally called regional citrate anticoagulation (RCA). The low iCa levels generated by infusion of citrate into the arterial line (the line between the vein from which blood is withdrawn and the dialyzer, *i*.*e*. upstream of the dialyzer) have to be raised in the venous line (*i.e.* the line downstream of the dialyzer via which the dialyzed blood is returned to the patient) before the blood re-enters the patient's systemic circulation. Citrate infusion and calcium infusion have to be balanced in order to avoid citrate toxicity and systemic hypo- or hyper-calcemia in the patient. This requires close monitoring, which is a labour intensive process.

US 2011/0237996 relates to a method of performing regional citrate anticoagulant dialysis of a patient's blood wherein a dialysis fluid is used containing calcium and citrate, but wherein also citrate is added to the patient's blood upstream of the dialyzer. A computer-controlled introduction of the citrate into the bloodstream is proposed. This invention claims as advantage as compared to RCA, that no separate infusion pump for iCa is required. However, it still needs a separate infusion pump for citrate which may cause citrate toxicity (the blood pump of the dialysis machine may have to be stopped during the dialysis procedure because of some technical problem with the patient or the machine; because of the simultaneous occurrence of alarms and the necessity to address the causes, stopping the separate citrate pump may easily be forgotten. After restarting the blood pump a high concentration of citrate will reach the patient).

DE 196 54 746 relates to a method wherein blood is infused with a citrate solution of 1-15 mmol/l, preferably 5-13 mmol/l, upstream of the dialyzer, which solution is characterized by a hydrogencarbonate concentration of 0-32 mMol/l. The solution to be used for the infusion is stated to be substantially free of calcium and magnesium, which according to claim 2 of this document may mean a concentration of about 0.2 mMol/l (calcium). Subsequent dialysis takes place in a dialysis bath which may contain 6 mMol/l citrate. In spite of the use of heparin as anticoagulant, the efficiency of dialysis can be compromised by the blockage of dialyzer membrane pores by clotted blood. Accordingly, there is a need in the art for improved dialysis fluid formulations that increase the efficiency of the hemodialysis treatment. Therefore a dialysis fluid containing citrate, but also calcium, magnesium and acetate made from a liquid concentrate (Citrasate®) or from it's dry equivalent (DRYalysate®), was tried (*cf.* USA Patent no US 6,610,206 B1). However, it has been reported that Citrasate without the concomitant use of heparin results in clotting of the dialyzer in two out of ten cases. (M. Dittrich et al. J Am Soc Nephrol 19 (2008), page 461A, abstract F-P01576;).

Annie Tu et al.: "Heparin-Free Hemodialysis with Citrate-Containing Dialysate in Intensive Care Patients", Dialysis and Transplantation, vol. 29, no. 10, 31 October 2000, pages 620-624 and Y.-L. Cheng et al.: "Anticoagulation during haemodialysis using a citrate-enriched dialysate: a feasibility study", Nephrology Dialysis Transplantation, vol. 26, no. 2, July 2010, pages 641-646, also discuss dialysis treatment using a citrate-containing dialysis fluid, containing a substantial amount of calcium (2.5 or 3.0 mEq/l in 'Tu et al." and 2.3 mEq/l in "Cheng et al."). These documents also report clotting of the system in a number of cases to the extent that treatment had to be abandoned.

There remains a need for alternative methods of hemodialysis or hemodiafiltration and for alternative products for use in such methods, in particular for alternatives that allow effective prevention of blood clotting during HD and HDF, wherein one or more problems such as mentioned above or described herein below are reduced or overcome.

It has now been found that hemodialysis or hemodiafiltration with a specific dialysis fluid in combination with a substitution fluid provides such an alternative.
Accordingly, the invention relates to a dialysis fluid containing citrate, the citrate concentration being in the range of more than 0.7 mmol/l and less than 2 mmol/l, which dialysis fluid is essentially free of calcium and essentially free of magnesium, wherein the term 'essentially free of means a concentration of less than 0.1 mmol/l, for use in a hemodialysis or hemodiafiltration therapy of blood without adding citrate or other anticoagulant to the blood, upstream of the dialyzer,
wherein the dialysis fluid is to be used for dialyzing or hemodiafiltering blood, which dialysed blood is to be mixed with a substitution fluid after which the dialyzed blood is to be returned to the subject undergoing the therapy, wherein the substitution fluid contains calcium.

Further, the invention relates to a dialysis fluid that is essentially free of calcium and essentially free of magnesium, the dialysis fluid containing
- 0.8 - 1.2 mmol/l citrate;
- 135 - 145 mmol/l sodium;
- 0 - 4.0 mmol/l potassium;
- 0 - 11 mmol/l glucose;
- 25 - 37 mmol/l bicarbonate;
   and chloride in a concentration to keep the electrochemical balance, , wherein the term 'essentially free of means a concentration of less than 0.1 mmol/l.

The invention further relates to a liquid or dry concentrate suitable for preparing a dialysis fluid according to the invention by diluting said liquid concentrate with water or respectively by reconstituting said dry concentrate in water), said concentrate containing
- 0.39 - 0.75 mol % citrate, in particular 0.40 - 0.60 mol % citrate;
- 67 - 88 mol % sodium, in particular 72 - 84 mol % sodium;
- 0 - 2.5 mol % potassium, in particular 0.5-2.0 mol % potassium;
- 0 - 7 mol % glucose, in particular 1 - 6 mol % glucose;
- 12 - 20 mol % bicarbonate, in particular 15 to 20 mol % bicarbonate,
   all based on the total of citrate, sodium, potassium, glucose, acetate, bicarbonate, and chloride in an amount to keep the electrochemical balance. The balance of the contents of the dialysis fluid is preferably water.

Also disclosed is a kit of parts suitable for preparing a dialysis fluid according to the invention, comprising (i) a first container containing a first concentrate, (ii) a second container containing a second concentrate different from the first concentrate. In an aspect the concentrates are liquids, to be diluted prior to use. In another aspect at least one of the concentrates is a dry product. In an advantageous aspect, the kit of parts comprises a (iii) a third container containing a third concentrate different from the first and from the second concentrate. In an advantageous aspect, said second container and/or third container are cartridges containing solid sodium bicarbonate salt (powder or granules) and sodium chloride salt (powder or granules) respectively.

The invention further relates to a kit of parts containing (i) a dialysis fluid according to the invention, or a concentrate for such a dialysis fluid according to the invention and (ii) a substitution fluid containing calcium and magnesium.

The invention further relates to a method for extracorporeally dialyzing or diafiltering blood, comprising
- dialyzing or diafiltering the blood against a dialysis fluid containing citrate, the citrate concentration being in the range of more than 0.7 mmol/l and 2 mmol or less, which dialysis fluid is essentially free of calcium and essentially free of magnesium; and
- mixing the dialyzed blood with a substitution fluid containing calcium and preferably magnesium,
   wherein the term essentially free means a concentration of less than 0.1 mmol/l.

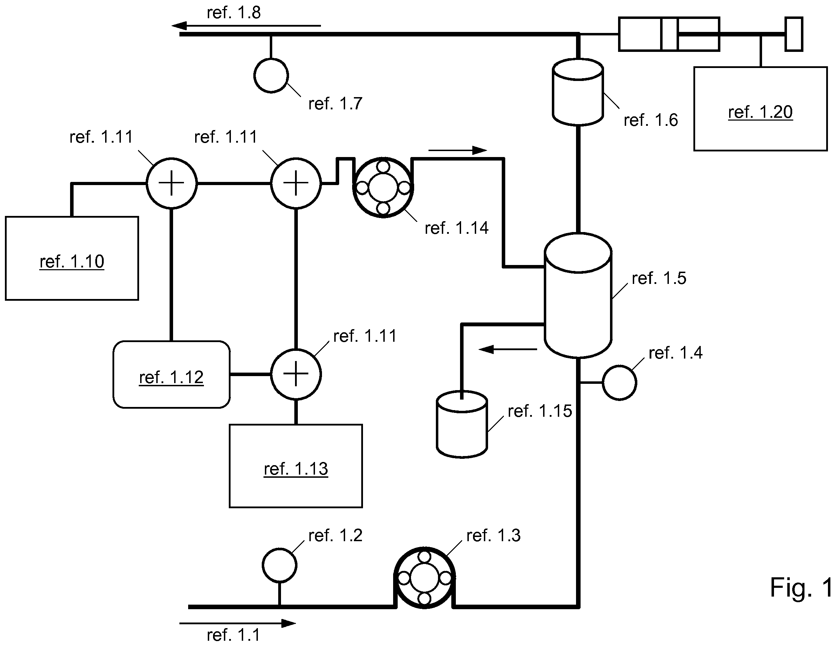

Figure 1 schematically shows how a dialysis fluid and a substitution fluid can be used for hemodialysis or hemodiafiltration.

The term "or" as used herein is defined as "and/or" unless specified otherwise.

The term "a" or "an" as used herein is defined as "at least one" unless specified otherwise.

When referring to a noun (*e.g.* a compound, an additive, *etc.*) in the singular, the plural is meant to be included

When referring herein to "essentially free", this generally means that a component is absent (not detectable), or -if present- in such a low level, typically present as a result of an impurity in the ingredients used for the preparation of a composition of the invention, and in such as concentration that it does not cause any significant undesired effect. In particular, a composition is considered essentially free of a component, if the concentration is less than 0.1 mmol/l, more in particular less than 0.01 mmol/l (in a liquid composition), and/or less than 0.1 wt.% of the dry weight of the composition, more in particular less than 0.01 wt.% of the dry weight (in a liquid composition or in a dry product, such as a dry concentrate).

As is generally known, an acid or base dissolved in water will form an acid-base equilibrium that is characteristic for a specific acid or base, dependent on their dissociation constant. For example, when trisodium citrate (Na₃C₆H₅O₇) is dissolved in an aqueous liquid an equilibrium will be formed between C₆H₅O₇³⁻, C₆H₆O₇²⁻, C₆H₇O₇¹⁻ and C₆H₈O₇ (fully protonated citric acid), in ratios that are specific for the existing pH in the liquid. Thus then referring herein to a carboxylate, such as citrate, dissolved in an aqueous liquid, this includes the carboxylate (citrate) that forms part of the fully protonated acid group (e.g. citric acid - C₆H₈O₇), as well as the partially (e.g. C₆H₆O₇²⁻, C₆H₇O₇¹⁻) or fully deprotonated group (*e*.*g*. C₆H₅O₇³⁻) as well as salts thereof. Likewise when a carboxylic acid is dissolved in an aqueous liquid it will form an equilibrium with the various conjugated bases of the carboxylic acid.

Reference herein is made to alkaline earth metal elements (calcium, magnesium) and to alkali metal elements (sodium, potassium). The names of those elements are in general spelled out herein in full ("calcium", " magnesium", " sodium" , or " potassium " respectively), when referring to the total amount/concentration of the respective elements in a composition (dialysis fluid, blood, substitution fluid, concentrate). The elements are typically present in an ionic form, such as a free ion (also referred to as ionized), as part of a complex or as part of a salt (in particular in a solid concentrate). The amount or concentration of an element can routinely be determined with a conventional technique such as atomic emission spectroscopy or atomic absorption spectroscopy.

The term 'ionized' as an adjective for a metal element, *i.c.* ionized calcium (iCa) and/or ionized magnesium (iMg) is used for calcium or magnesium that is available for forming a complex with citrate, in particular in blood. The concentration of iCa or iMg can routinely be determined, also in a complex fluid such as blood, for instance by using atomic emission spectrometry. For iCa a blood-gas analyzer. such as the ABL-800 is particularly suitable. As a general indication, in blood 50-80 % of calcium is bound to a protein or a small complexing agent (such as citrate). 20-50 % of calcium is present as iCa. In accordance with the present invention, the term 'hemodialysis' (HD) is used for a technique wherein blood is extracorporeally subjected to a treatment wherein toxins, metabolites, ions in the blood are allowed to diffuse into a fluid (the dialysis fluid) that is separated from the blood via a semi-permeable membrane. The diffusion takes place in a dialyzer, a device which is also referred to in the art as an artificial kidney, as it removes toxins that are typically removed from the blood by the kidneys in a healthy person. Hemodialysis is thus a kind of renal replacement therapy.

Hemodiafiltration can be regarded as a combination of diffusion and filtration. Blood is pumped through the blood compartment of a dialyzer and an increased transmembrane pressure is used to filter plasma fluid. The dialyzer, usually a high flux dialyzer, is used to filter plasma fluid from the blood compartment to the dialysate compartment. The loss of plasma fluid in the blood compartment then will be replaced by the dialysis fluid that is infused directly into the blood line. At the same time dialysis fluid is delivered to the dialysate compartment of the dialyzer as in HD. The combination of diffusion and filtration is in particular useful because it results in good removal of both large and small molecular weight solutes.

As used herein, 'sterile' means in particular that the microbiological quality is satisfactory for the intended use. For hemodialysis the dialysis fluid is sterile if it meets the requirements of the European Pharmacopee 7thEdition Year, 2013, as available from ht.t.p://online.pheur.org/entry.htm. (Monography "Solutions for hemofiltration and for hemodiafiltration").

Advantageously, the HD or HDF in accordance with the invention is directed to the combined use of the dialysis fluid and the substitution fluid.

The dialysis fluid contains citrate. The dialysis fluid is essentially free of calcium, and essentially free of magnesium. The dialysisis fluid is preferably free of divalent metal ions.

In principle, the dialysis fluid may contain one or more other organic acids, such as acetate. In an embodiment, the dialysis fluid contains 0-1 mmol/l acetate. In an embodiment, the concentrate contains 0-0.7 mol % acetate. In an advantageous embodiment, the dialysis fluid is essentially free of acetate and - preferably essentially free of other organic acids except citrate (present as free acid or as one of its conjugated bases; *i*.*e*. as carboxylate anion).

The substitution fluid contains calcium (iCa), and preferably magnesium (iMg),.

The substitution fluid is usually essentially free of acetate, citrate and other organic acids. Preferably, the substitution fluid is a solution essentially consisting of a calcium salt and - if desired - a magnesium salt in water. In an advantageous embodiment, the substitution fluid consists of a calcium chloride solution in water or a solution of calcium chloride and magnesium chloride in water.

Surprisingly, the combination of the dialysis fluid and the substitution fluid (for use) in accordance with the invention enables adequate anticoagulation in the extracorporeal dialysis circuit while the post-dialyzer divalent cation infusion (calcium infusion and preferably magnesium infusion, using the substitution fluid) enables the clinician to custom tailor patient's calcium metabolism. The fact that the dialysis fluid is free of calcium and free of magnesium obviates the need for acetate in the dialysis fluid, which is claimed to cause hemodynamic instability.

It is in particular also an advantage of the invention that no citrate or other anticoagulant needs to be added to the blood, upstream of the dialyzer and that the citrate in the dialysis fluid suffices to avoid coagulation.

Citrate toxicity may occur when the blood levels of citrate in the patient are above 2.5 mmol/l. In the present invention a substantial risk of reaching such levels is generally avoided, because no separate pump is used to infuse citrate into the extracorporeal dialysis circuit. The dialysis fluid can be the only citrate source in the HD or HDF process, and a citrate concentration below 2.0 mmol/l is typically sufficient for effective extracorporeal anticoagulation. By its design, the invention prevents blood levels of citrate of >2.0 mmol/l. Such a low concentration is generally considered to be non-toxic, as it is generally sufficiently low to allow metabolization in skeletal muscle and liver tissue. Only in cases of severe hepatic failure combined with severe shock, or of certain (rare) metabolic diseases, the metabolism of citrate may run short leading to too high blood levels of citrate. The amount of calcium and magnesium migrating into the dialysis fluid is positively correlated with the levels of iCa and iMg in the blood entering the dialyzer and with the blood- and dialysis fluid flow. The loss of these divalent cations has to be substituted in the patient's blood downstream of the dialyzer. The risk of substituting too much of the divalent cations is minimized by the positive correlation between the blood levels and loss over the semi-permeable membrane. The risk of substituting not enough of the divalent cations with the advised schedules is in particular overcome by measuring the blood level of iCa in the arterial line (reflecting the iCa concentration as existing in the patient) pre-dialysis and about 2 hours after the start of the first HD or HDF session. The substitution rate should be increased if 2 hours after the start, the iCa level is decreased as compared to pre-dialysis levels by 10% or more and if 2 hours after the start iCa level is below the normal value. If both prerequisites are fulfilled the substitution rate should be increased by 10% and a post-dialysis iCa measurement performed. If only one of the 2 prerequisites is fulfilled the substitution rate should be increased by 5% and a post-dialysis iCa measurement performed.

Thus, this invention offers many advantages, including potentially eliminating well-known downsides of heparin anticoagulation (such as increased bleeding risk, and heparin drug side-effects), and addressing critical shortcomings of classic citrate anticoagulation. For example, no separate citrate infusion is required, so no need for a separate infusion pump, which would make citrate anticoagulation less laborious compared to current state of the art citrate anticoagulation.

Also, a separate pump with citrate produces the risk of accumulation of citrate in case of stopping the blood pump of the dialysis machine. During dialysis the blood pump may have to be stopped temporarily due to technical problems of the dialysis procedure. If the separate infusion pump with citrate is not stopped simultaneously, a flush of concentrated citrate will be infused, when the blood pump is restarted. This may cause citrate toxicity in the patient. The invention prevents this accumulation of citrate.

The frequent monitoring of systemic iCa and subsequent adjustments of the calcium infusion rate is reduced to the first HD or HDF session for a specific patient only and typically comprises to the maximum 3 samples. This invention eliminates the danger in the potential for equipment failure and user error, resulting in improved patient safety.

The dialysis fluid (for use) according to the invention is generally used in combination with a substitution fluid, wherein the dialysis fluid is to be used for hemodialyzing or hemodiafiltering blood, which dialyzed blood is to be mixed with the substitution fluid after which the dialyzed blood is to be returned to the subject undergoing the therapy, wherein the substitution fluid contains calcium. In case of hemodiafiltering blood, the plasma fluid loss is to be replaced by same dialysis fluid. The dialysis fluid is an aqueous solution of citrate and usually one or more other components. The dialysis fluid is usually essentially free of solvents other than water.

In a particularly preferred embodiment, the dialysis fluid is essentially free of divalent cations or other cations that are capable of forming a complex with citrate, thereby reducing the effectivity of citrate.

Generally a substitution fluid is added to the dialyzed or diafiltered blood that contains magnesium. The amount of citrate in the dialysis fluid is generally chosen such that it is sufficient to anticoagulate patient's blood in the extracorporeal dialysis circuit.

The citrate concentration is more than 0.7 mmol/l. The citrate concentration preferably is 0.8 mmol/l or more.

The citrate concentration may be 0.9 mmol/l or more, or 1.0 mmol/l or more.

The citrate concentration in the dialysis fluid for use according to the invention or used in a method according to the invention is 2 mmol/l or less, in particular 1.5 mmol/l or less.

Usually, the dialysis fluid also contains sodium, generally in a concentration of more than 100 mmol/l. Preferably the sodium concentration is at least 135 mmol/l. The sodium concentration is usually less than 150 mmol, in particular 145 mmol/l or less.

Optionally, the dialysis fluid comprises potassium. If present, the concentration preferably is at least 0.5 mmol/l, in particular at least 1.0 mmol/l. The potassium concentration usually is 4 mmol/l or less.

Optionally glucose is present. Usually, no other carbohydrates are present. The total carbohydrate concentration, in particular the glucose concentration, is usually 11 mmol/l or less. If present, the glucose concentration in the dialysis fluid is usually at least 1 mmol/l.

Usually, the dialysis fluid comprises bicarbonate. Usually, the bicarbonate concentration is more than 20 mmol/l, preferably at least 25 mmol/l. The bicarbonate concentration is usually less than 50 mmol/l, preferably 40 mmol/l or less.

The dialysis fluid usually also contains chloride. Chloride is typically present in a concentration effective to keep the electrochemical balance.

The pH of the dialysis fluid is usually in the range of 7 to 9 (as measured with a standard pH electrode at 25 °C).

The dialysis fluid is typically essentially free of heparin, and preferably essentially free of any anticoagulant other than citrate.

Usually, the dialysis fluid is essentially free of phosphate. However, in a specific embodiment, the dialysis fluid contains phosphate. The presence of phosphate is in particular useful in case the dialysis fluid is intended for night-dialysis or permanent (24-hours) dialysis.

The dialysis fluid is preferably sterile, at least when used for the dialysis. Preferably the dialysis fluid, respectively a concentrate for the dialysis fluid, is packaged sterile, but in principle the dialysis fluid may also be sterilised shortly or immediately before use.

In an embodiment, the dialysis fluid is prepared by dissolving citric acid or a citrate salt, preferably tri sodium citrate, and the other components that may be present, such as bicarbonate, preferably sodium bicarbonate, potassium (as citrate salt, chloride salt or bicarbonate salt) in water. If the pH is higher than desired, the pH may be adjusted with an acid, preferably chloric acid (HCl) or citric acid (C₆H₈O₇); if the pH is lower than desired the pH may be adjusted with a base, preferably trisodium citrate, bisodium carbonate or sodium hydroxide .

In an advantageous embodiment, the dialysis fluid is made by mixing two or more concentrates, each comprising a part of the required ingredients for the dialysis fluid.

The mixing of the concentrates may be performed isolated from the HD or HDF or may be performed continuously in mixing units, forming part of the HD or HDF system (see Figure 1). Other components of the dialysis fluid may be present in either one of or both of the concentrates.

In advantageous embodiment, at least one of said concentrates is a liquid and at least one concentrate is a solid such as a particulate (granular or powdered) solid. In such embodiment, preferably, a concentrate containing bicarbonate is the solid concentrate. A solid concentrate is advantageously housed in a cartridge through which water is allowed to flow during use, resulting in dissolution of the solid concentrate in the water, and wherein the resultant solution is to be mixed with the liquid concentrate. Such cartridges are e.g. known from Gambro. In case the dialysis fluid is prepared from three concentrates, the third one can also be a solid such as a particulate (granular or powdered) solid. A solid concentrate preferably comprises only one ingredient, such as only bicarbonate, only NaCl or only citrate.

This is considered advantageous in view of well-controlled dissolution of the solid concentrate.

In an embodiment a first concentrate, containing citrate, preferably trisodium citrate, and a second concentrate, containing bicarbonate, preferably sodium bicarbonate are mixed. Optionally, the second concentrate also comprises citrate. Any other ingredients may be present in any of the concentrates. In a specific embodiment wherein the dialysis fluid is to be prepared from two concentrates, the first concentrate at least substantially consists of a solid bicarbonate and the second concentrate consists of citrate and any optional components other than bicarbonate.

In a further embodiment, a first concentrate comprises bicarbonate, a second concentrate comprises NaCl and citrate plus any optional components are each independently present in the first concentrate, the second concentrate or in both.

In a further embodiment, a first concentrate comprises bicarbonate, a second concentrate comprises NaCl, and a third concentrate comprises citrate plus any optional components. In such embodiment, the first and second concentrate are advantageously solid (such as solid particles) whereas the third concentrate is a liquid concentrate. The solid concentrates are typically separately dissolved in water and the resultant solutions are mixed with the liquid concentrate.

The calcium concentration in the substitution fluid is usually at least 350 mmol/l, preferably at least 500 mmol/l. The calcium concentration usually is less than 2200 mmol/l, in particular 1600 mmol/l or less.

If present, the magnesium concentration in the substitution fluid is usually at least 180 mmol/l, preferably at least 220 mmol/l. The magnesium concentration usually is less than 1000 mmol/l, in particular 500 mmol/l or less.

The pH of the substitution fluid is usually in the range of 6-7. The substitution fluid is usually sterile.

Hemodialysis or hemodiafiltration in relation to the present invention, is generally directed to the combined use of (i) the dialysis fluid containing citrate, yet essentially free of calcium, and magnesium, with (ii) the substitution infusion fluid containing calcium, and preferably magnesium (both divalent cations).

The hemodialysis or hemodiafiltration in accordance with the invention is suitable for the treatment of any kind of blood, in particular human blood. The blood is usually from a patient suffering from renal failure. Hemodialysis or hemodiafiltration in accordance with the invention is in particular advantageous for treatment of blood from a human with renal failure and a risk of bleeding due to the concomitant use of anticoagulants (e.g. vitamin K antagonists, thrombin inhibitors or prostaglandin inhibitors), gastrointestinal lesions (e.g. gastritis, peptic ulcer, and angiodysplasia), recent surgery, or pericarditis. The blood may also be from a human suffering from renal failure with a risk for developing cerebral microbleeds.

Usually, in the HD or HDF, blood is pumped through an extracorporeal circuit with a dialyzer. The dialyzer is divided by a semi-permeable membrane in a blood compartment and a dialysis fluid compartment. In the blood compartment the blood flow is preferably in countercurrent to the dialysis fluid flow in the dialysis fluid compartment, although concurrent flow is also possible.

During HD or HDF citrate diffuses into the patient's blood whereas calcium (and magnesium if the iMg concentration in the blood is higher than in the dialysis fluid, which generally is the case) is (are) diffusing into the dialysis fluid. Such an exchange of citrate, calcium (and magnesium) in the dialyzer results in an effective anticoagulation of the patient's blood in the extracorporeal dialysis circuit, wherein the patient's blood is dialyzed.

The amount of calcium, respectively magnesium, lost in the dialysis fluid is positively correlated with the levels of iCa, respectively iMg, in the blood entering the dialyzer and with the blood flow and the dialysis fluid flow. The loss of these divalent cations has to be substituted in the patient's blood downstream of the dialyzer. The risk of substituting too much of the divalent cations is minimized by the aforementioned positive correlation between the blood levels and loss over the semi-permeable membrane. The risk of substituting not enough of the divalent cations with the advised schedules is overcome by measuring the blood level of iCa in the arterial line pre-dialysis and 2 hours after the start of the first HD or HDF session. The substitution rate should be increased if 2 hours after the start iCa levels were decreased as compared to pre-dialysis levels by 10% or more and if 2 hours after the start iCa level was below the normal value. If both prerequisites are fulfilled the substitution rate should be increased by 10% and a post-dialysis iCa measurement performed. In particular for HD it is preferred that the substitution rate is increased by 5% and a post-dialysis iCa measurement performed, if only one of the two prerequisites is fulfilled.

In a preferred embodiment, the dialysis fluid is for use in combination with the substitution fluid, wherein the rate of administering the substitution fluid is determined based on the blood flow rate of the dialyzed blood and the calcium homeostasis, and optionally the phosphate homeostasis of the subject undergoing the therapy

This can be accomplished in various ways, and the skilled will understand how to carry this out based on common general knowledge and the information disclosed herein.

For instance, in case patient's calcium homeostasis is well controlled with a conventional dialysis fluid containing Ca 1.25 mmol/l the rate of administering the substitution infusion fluid containing Ca into the patient's blood is according to following schedule, which is in particular suitable for HD:

| | |
|---|---|
| blood flow in the extracorporeal circuit >200 ml/min | Ca 0.20 mmol/min |
| blood flow in the extracorporeal circuit 150 ml/min | Ca 0.16 mmol/min |
| blood flow in the extracorporeal circuit 100 ml/min | Ca 0.13 mmol/min |

Alternatively, in case patient's calcium homeostasis is well controlled with a conventional dialysis fluid containing Ca 1.5 mmol/l the rate of administering the substitution infusion fluid containing Ca into the patient's blood is according to following schedule:

| | |
|---|---|
| blood flow in the extracorporeal circuit >200 ml/min | Ca 0.22 mmol/min |
| blood flow in the extracorporeal circuit 150 ml/min | Ca 0.18 mmol/min |
| blood flow in the extracorporeal circuit 100 ml/min | Ca 0.15 mmol/min |

Alternatively, in case patient's calcium homeostasis is well controlled with a conventional dialysis fluid containing Ca 1.75 mmol/l the rate of administering the substitution infusion fluid containing Ca into the patient's blood is according to following schedule:

| | |
|---|---|
| blood flow in the extracorporeal circuit >200 ml/min | Ca 0.24 mmol/min |
| blood flow in the extracorporeal circuit 150 ml/min | Ca 0.20 mmol/min |
| blood flow in the extracorporeal circuit 100 ml/min | Ca 0.17 mmol/min. |

By way of example, the invention will now be described in more detail while referring to the Figure 1, which illustrates an HD process, making use of two concentrates for preparing the dialysis fluid. On the basis of the present disclosure, common general knowledge, and known equipment (commercially available e.g. from Gambro or Fresenius) the skilled person will also be able to reduce other embodiments of the invention to practice.

Blood is extracted from a blood circulation of the patient and transported to a dialyzer via a blood pump in the so-called arterial line of an extracorporeal circuit. The pressure in the arterial line before the blood pump is registered by a pressure sensor. Increased pressure in the arterial line may indicate clot formation in the extracorporeal circuit. In the dialyzer, the blood is passing along a semi-permeable membrane. On the other side of the semi-permeable membrane, the dialysis fluid flows in the opposite direction *i*.*e*. counter-current of the blood. In case a patient starts HD and HDF, the dialysis fluid flow may be concurrent during the first couple of sessions.

Figure 1 schematically shows a system for carrying out a haemodialysis in accordance with the invention.

### Reference list to figure 1

- ref. 1.1= Blood from patient
- ref. 1.2= Arterial pressure sensor
- ref. 1.3= Blood pump
- ref. 1.4= Blood pressure sensor
- ref. 1.5= Dialyser
- ref. 1.6= Air trap air sensor
- ref. 1.7= Venous pressure sensor
- ref. 1.8= Blood to patient
- ref. 1.10= Citrate containing concentrate and delivery mechanism
- ref. 1.11= Mixing valve
- ref. 1.12= Water
- ref. 1.13= Bicarbonate cartridge and delivery mechanism
- ref. 1.14= Dialysate pump
- ref. 1.15= Used dialysate
- ref. 1.20= Divalent cations containing substitution fluid

As shown in the Figure 1, the dialysis fluid may be prepared by mixing via a valve a bicarbonate solution with a citrate solution.

The mixing valve assures that a dialysis fluid with a specific conductivity and temperature is delivered to the dialysis fluid pump, which determines the dialysis fluid flow rate, which is mostly 500 ml/min. The bicarbonate solution is prepared by adding water to a bicarbonate cartridge and then transported via a mixing valve to the citrate containing solution. This latter solution is prepared by diluting a citrate containing concentrate via a mixing valve with water. After passing the dialyzer, the dialysis fluid is called dialysate.

After the dialyzer the blood preferably passes an air trap and thereafter the divalent cations containing substitution infusion fluid is added to the blood in the so-called venous line. Usually, the pressure in the venous line is registered as well and a drop in this pressure may indicate clot formation in the extracorporeal circuit.

### Examples

### Example 1: kit of parts containing two concentrates

The first concentrate is an aqueous concentrate comprising:
between 36 and 54 mmol/L of citrate;
between 4500 and 4700 mmol/L of sodium;
between 45 and 135 mmol/L of potassium;
between 135 and 500 mmol/L of glucose;
and chloride ions to keep electrochemical balance.

The second concentrate is an aqueous solution comprising between 600 and 1000 mmol/l sodium bicarbonate.

The first concentrate, the second concentrate and water can be mixed in a ratio of 1 to about 1.75 to about 42.25 to obtain a dialysis fluid suitable for use in accordance with the invention.

### Example 2: kit of parts containing three concentrates

The first concentrate is a sodium, potassium, glucose and chloride containing aqueous concentrate comprising:
between 100 and 500 mmol/L of citrate;
between 0 and 1500 mmol/L of sodium;
between 0 and 1500 mmol/L of potassium;
between 300 and 5500 mmol/L of glucose and chloride ions to keep electrochemical balance.

This concentrate is in particular suitable for mixing with a sodium bicarbonate solution prepared from a cartridge (second concentrate, free of citrate) and a sodium chloride solution prepared from a cartridge (third concentrate, free of citrate). The first concentrate can be mixed with the solution containing bicarbonate (34 mmol/l), sodium (140 mmol/l) and water in a volume ratio of 1: 200 or 1:400 to obtain a dialysis fluid suitable for use in accordance with the invention.

### Example 3: kit of parts containing two concentrates

The first concentrate comprises:
between 4500 and 4700 mmol/L of sodium;
between 45 and 135 mmol/L of potassium;
between 135 and 500 mmol/L of glucose and chloride ions to keep electrochemical balance.

The second concentrate (sodium bicarbonate containing concentrate) comprises:
between 21 and 31 mmol/L citrate
between 600 and 1000 mmol/l sodium bicarbonate

These concentrates are in particular suitable for mixing in a ratio of first concentrate : second concentrate : water in a volume ratio of 1: about 1.75 : about 40 (*e.g.* 1:1.755: 42.245) to obtain a dialysis fluid suitable for use according to the invention.

## Claims

1. A dialysis fluid containing citrate, the citrate concentration being in the range of more than 0.7 mmol/l and less than 2 mmol/l, which dialysis fluid is essentially free of calcium and essentially free of magnesium, wherein the term 'essentially free of means a concentration of less than 0.1 mmol/l, for use in a hemodialysis therapy or hemodiafiltration therapy of blood without adding citrate or other anticoagulant to the blood, upstream of the dialyzer,
wherein the dialysis fluid is to be used for dialyzing or hemodiafiltering blood, which dialysed blood is to be mixed with a substitution fluid after which the dialyzed blood is to be returned to the subject undergoing the therapy, wherein the substitution fluid contains calcium.

2. Dialysis fluid for use according to claim 1, wherein the substitution fluid contains calcium and magnesium.

3. Dialysis fluid for use according to claim 2, wherein the substitution fluid contains 500-1600 mmol/l calcium and 220-500 mmol/l magnesium.

4. Dialysis fluid for use according to any of the claims 1-3, wherein the dialysis fluid is essentially free of acetate, i.e. wherein the acetate concentration is less than 0.1 mmol/l.

5. Dialysis fluid for use according to any of the claims 1-4, wherein the dialysis fluid contains 0.8-1.2 mmol/l citrate.

6. Dialysis fluid for use according to any of the claims 1-5, wherein the dialysis fluid contains
- 135-145 mmol/l sodium, 0-4.0 mmol/l potassium, 0-11 mmol/l glucose,
25-37 mmol/l bicarbonate, and chloride in a concentration to keep the electrochemical balance.

7. Dialysis fluid for use according to any of the claims 1-6, wherein the dialysis fluid is a mixture made from a first concentrate, a second concentrate, wherein citrate is present in the first concentrate, the second concentrate, in both the first and the second concentrate, or in a third concentrate.

8. Dialysis fluid for use according to any of the claims 1-7, wherein the rate of administering the substitution fluid is determined based on the blood flow rate of the dialyzed blood and the calcium homeostasis of the subject undergoing the therapy.

9. Dialysis fluid for use according to any of the preceding claims, wherein the calcium concentration in the dialysis fluid is less than 0,01 mmol/l.

10. Dialysis fluid for use according to claim 9, wherein calcium and/or magnesium are absent in the dialysis fluid.

11. Dialysis fluid, that is essentially free of calcium and essentially free of magnesium, the dialysis fluid containing
- 0.8 - 1.2 mmol/l citrate;
- 135 - 145 mmol/l sodium;
- 0- 4.0 mmol/l potassium;
- 0 - 11 mmol/l glucose;
- 25 - 37 mmol/l bicarbonate;
and chloride in a concentration to keep the electrochemical balance, wherein the term 'essentially free of means a concentration of less than 0.1 mmol/l.

12. Dialysis fluid according to claim 11, wherein the citrate concentration is 0.8 mmol/l citrate.

13. Liquid or dry concentrate suitable for preparing a dialysis fluid according to claim 11 or 12 by diluting said liquid concentrate with water or respectively by reconstituting said dry concentrate in water, said concentrate containing
- 0.39 - 0.75 mol % citrate, in particular 0.40 - 0.6 mol % citrate;
- 67 -88 mol % sodium, in particular 72 - 84 mol % sodium;
- 0 - 2.5 mol % potassium, in particular 0.5-2.0 mol % potassium;
- 0 - 7 mol % glucose, in particular 1 - 6 mol % glucose,
- 12 - 20 mol % bicarbonate, in particular 15 to 20 mol % bicarbonate; all based on the total of citrate, sodium, potassium, glucose and bicarbonate , and chloride in an amount to keep the electrochemical balance.

14. Kit of parts containing (i) a dialysis fluid according to claim 11 or 12 - or a concentrate according to claim 13 and (ii) a substitution fluid, containing calcium, or a concentrate for such a substitution fluid.

15. Method for extracorporeally dialyzing or diafiltering blood, comprising
- dialyzing or diafiltering the blood against a dialysis fluid containing citrate, the citrate concentration being in the range of more than 0.7 mmol/l and 2 mmol/l or less, which dialysis fluid is essentially free of calcium and essentially free of magnesium, without adding citrate or other anticoagulant to the blood, upstream of the dialyzer; and
- mixing the dialyzed blood with a substitution fluid containing calcium and preferably magnesium,
wherein the term essentially free means a concentration of less than 0.1 mmol/l.

## Patentansprüche

1. Dialyseflüssigkeit enthaltend Citrat, wobei die Citratkonzentration in dem Bereich von mehr als 0,7 mmol/l und weniger als 2 mmol/l ist, wobei die Dialyseflüssigkeit im Wesentlichen frei von Calcium und im Wesentlichen frei von Magnesium ist, wobei der Ausdruck "im Wesentlichen frei von" eine Konzentration von weniger als 0,1 mmol/l bedeutet, zur Verwendung in einer Hämodialysetherapie oder Hämodiafiltrationstherapie von Blut ohne Hinzufügen von Citrat oder anderem Antikoagulans zu dem Blut, stromaufwärts des Dialysators, wobei die Dialyseflüssigkeit für die Dialyse oder Hämodiafiltration von Blut verwendet werden soll, wobei das dialysierte Blut mit einer Substitutionsflüssigkeit gemischt werden soll, wonach das dialysierte Blut zu dem sich der Therapie unterziehenden Subjekt zurückgeführt werden soll, wobei die Substitutionsflüssigkeit Calcium enthält.

2. Dialyseflüssigkeit zur Verwendung nach Anspruch 1, wobei die Substitutionsflüssigkeit Calcium und Magnesium enthält.

3. Dialyseflüssigkeit zur Verwendung nach Anspruch 2, wobei die Substitutionsflüssigkeit 500-1600 mmol/l Calcium und 220-500 mmol/l Magnesium enthält.

4. Dialyseflüssigkeit zur Verwendung nach einem der Ansprüche 1-3, wobei die Dialyseflüssigkeit im Wesentlichen frei von Acetat ist, d. h. wobei die Acetatkonzentration weniger als 0,1 mmol/l ist.

5. Dialyseflüssigkeit zur Verwendung nach einem der Ansprüche 1- 4, wobei die Dialyseflüssigkeit 0,8-1,2 mmol/l Citrat enthält.

6. Dialyseflüssigkeit zur Verwendung nach einem der Ansprüche 1- 5, wobei die Dialyseflüssigkeit enthält
- 135-145 mmol/l Natrium, 0-4,0 mmol/l Kalium, 0-11 mmol/l Glukose, 25-37 mmol/l Bikarbonat, und Chlorid in einer Konzentration um das elektrochemische Gleichgewicht zu halten.

7. Dialyseflüssigkeit zur Verwendung nach einem der Ansprüche 1- 6, wobei die Dialyseflüssigkeit eine Mischung ist, hergestellt aus einem ersten Konzentrat, einem zweiten Konzentrat, wobei Citrat in dem ersten Konzentrat, dem zweiten Konzentrat, in sowohl dem ersten als auch dem zweiten Konzentrat, oder in einem dritten Konzentrat vorhanden ist.

8. Dialyseflüssigkeit zur Verwendung nach einem der Ansprüche 1-7, wobei die Verabreichungsrate der Substitutionsflüssigkeit basierend auf der Blutflussrate des dialysierten Blutes und der Kalziumhomöostase des sich der Therapie unterziehenden Subjekts bestimmt wird.

9. Dialyseflüssigkeit zur Verwendung nach einem der vorhergehenden Ansprüche, wobei die Calciumkonzentration in der Dialyseflüssigkeit weniger als 0,01 mmol/l ist.

10. Dialyseflüssigkeit zur Verwendung nach Anspruch 9, wobei Calcium und/oder Magnesium in der Dialyseflüssigkeit abwesend sind.

11. Dialyseflüssigkeit, die im Wesentlichen frei von Calcium und im Wesentlichen frei von Magnesium ist, die Dialyseflüssigkeit enthaltend
- 0,8 - 1,2 mmol/l Citrat;
- 135 - 145 mmol/l Natrium;
- 0 - 4,0 mmol/l Kalium;
- 0 - 11 mmol/l Glukose;
- 25 - 37 mmol/l Bikarbonat;
und Chlorid in einer Konzentration um das elektrochemische Gleichgewicht zu halten, wobei der Ausdruck "im Wesentlichen frei von" eine Konzentration von weniger als 0,1 mmol/l bedeutet.

12. Dialyseflüssigkeit nach Anspruch 11, wobei die Citratkonzentration 0,8 mmol/l Citrat ist.

13. Flüssiges oder trockenes Konzentrat geeignet zum Zubereiten einer Dialyseflüssigkeit nach Anspruch 11 oder 12 durch Verdünnen des flüssigen Konzentrats mit Wasser oder entsprechend durch Rekonstituieren des trockenen Konzentrats in Wasser, das Konzentrat enthaltend
- 0,39 - 0,75 Mol-% Citrat, insbesondere 0,40 - 0,6 Mol-% Citrat;
- 67 - 88 Mol-% Natrium, insbesondere 72 - 84 Mol-% Natrium;
- 0 - 2,5 Mol-% Kalium, insbesondere 0,5-2,0 Mol-% Kalium;
- 0 - 7 Mol-% Glukose, insbesondere 1 - 6 Mol-% Glukose,
- 12 - 20 Mol-% Bicarbonat, insbesondere 15 bis 20 Mol-% Bikarbonat; alle basierend auf der Summe aus Citrat, Natrium, Kalium, Glukose und Bicarbonat, und Chlorid in einer Menge, um das elektrochemische Gleichgewicht zu halten.

14. Kit aus Teilen enthaltend (i) eine Dialyseflüssigkeit nach Anspruch 11 oder 12
- oder ein Konzentrat nach Anspruch 13 und (ii) eine Substitutionsflüssigkeit enthaltend Calcium, oder ein Konzentrat für eine solche Substitutionsflüssigkeit.

15. Verfahren zum extrakorporalen Dialysieren oder Diafiltrieren von Blut, umfassend
- Dialysieren oder Diafiltrieren des Blutes gegen eine Dialyseflüssigkeit enthaltend Citrat, wobei die Citratkonzentration in dem Bereich von mehr als 0,7 mmol/l und 2 mmol/l oder weniger ist, wobei die Dialyseflüssigkeit im Wesentlichen frei von Calcium und im Wesentlichen frei von Magnesium ist, ohne Hinzufügen von Citrat oder anderem Antikoagulans zum Blut, stromaufwärts des Dialysators; und
- Mischen des dialysierten Blutes mit einer Substitutionsflüssigkeit enthaltend Calcium und vorzugsweise Magnesium,
wobei der Ausdruck "im Wesentlichen frei" eine Konzentration von weniger als 0,1 mmol/l bedeutet.

## Revendications

1. Liquide de dialyse contenant du citrate, la concentration de citrate étant dans la plage de plus de 0,7 mmol/l et moins de 2 mmol/l, ledit liquide de dialyse étant essentiellement exempt de calcium et essentiellement exempt de magnésium, où le terme essentiellement exempt désigne une concentration inférieure à 0,1 mmol/l, pour utilisation dans un traitement d'hémodialyse ou un traitement d'hémodiafiltration de sang sans ajout de citrate ou d'un autre anticoagulant au sang, en amont du dialyseur,
où le liquide de dialyse est destiné à être utilisé pour dialyser ou hémodiafiltrer du sang, ledit sang dialysé étant destiné à être mélangé avec un liquide de substitution après quoi le sang dialysé est destiné à être retourné au sujet recevant le traitement, dans lequel le liquide de substitution contient du calcium.

2. Liquide de dialyse pour utilisation selon la revendication 1, dans lequel le liquide de substitution contient du calcium et du magnésium.

3. Liquide de dialyse pour utilisation selon la revendication 2, dans lequel le liquide de substitution contient 500 à 1600 mmol/l de calcium et 220 à 500 mmol/l de magnésium.

4. Liquide de dialyse pour utilisation selon l'une quelconque des revendications 1 à 3, où le liquide de dialyse est essentiellement exempt d'acétate, c'est-à-dire dans lequel la concentration d'acétate est inférieure à 0,1 mmol/l.

5. Liquide de dialyse pour utilisation selon l'une quelconque des revendications 1 à 4, où le liquide de dialyse contient 0,8 à 1,2 mmol/l de citrate.

6. Liquide de dialyse pour utilisation selon l'une quelconque des revendications 1 à 5, où le liquide de dialyse contient
- 135 à 145 mmol/l de sodium, 0 à 4,0 mmol/l de potassium, 0 à 11 mmol/l de glucose,
- 25 à 37 mmol/l de bicarbonate, et du chlorure à une concentration permettant de maintenir l'équilibre électrochimique.

7. Liquide de dialyse pour utilisation selon l'une quelconque des revendications 1 à 6, où le liquide de dialyse est un mélange constitué d'un premier concentré, d'un deuxième concentré, dans lequel le citrate est présent dans le premier concentré, le deuxième concentré, à la fois dans le premier et le deuxième concentré, ou dans un troisième concentré.

8. Liquide de dialyse pour utilisation selon l'une quelconque des revendications 1 à 7, où le débit d'administration du liquide de substitution est déterminé sur la base du débit sanguin du sang dialysé et de l'homéostasie calcique du sujet recevant le traitement.

9. Liquide de dialyse pour utilisation selon l'une quelconque des revendications précédentes, dans lequel la concentration de calcium dans le liquide de dialyse est inférieure à 0,01 mmol/l.

10. Liquide de dialyse pour utilisation selon la revendication 9, dans lequel le calcium et/ou le magnésium sont absents dans le liquide de dialyse.

11. Liquide de dialyse, qui est essentiellement exempt de calcium et essentiellement exempt de magnésium, le liquide de dialyse contenant
- 0,8 à 1,2 mmol/l de citrate ;
- 135 à 145 mmol/l de sodium ;
- 0 à 4,0 mmol/l de potassium ;
- 0 à 11 mmol/l de glucose ;
- 25 à 37 mmol/l de bicarbonate ;
et du chlorure à une concentration permettant de maintenir l'équilibre électrochimique, où le terme essentiellement exempt désigne une concentration inférieure à 0,1 mmol/l.

12. Liquide de dialyse selon la revendication 11, dans lequel la concentration de citrate est 0,8 mmol/l de citrate.

13. Concentré liquide ou sec adapté pour préparer un liquide de dialyse selon la revendication 11 ou 12 par dilution dudit concentré liquide avec de l'eau ou respectivement par reconstitution dudit concentré sec dans de l'eau, ledit concentré contenant
- 0,39 à 0,75 % en moles de citrate, en particulier 0,40 à 0,6 % en moles de citrate ;
- 67 à 88 % en moles de sodium, en particulier 72 à 84 % en moles de sodium ;
- 0 à 2,5 % en moles de potassium, en particulier 0,5 à 2,0 % en moles de potassium ;
- 0 à 7 % en moles de glucose, en particulier 1 à 6 % en moles de glucose,
- 12 à 20 % en moles de bicarbonate, en particulier 15 à 20 % en moles de bicarbonate ; tous sur la base du total des citrate, sodium, potassium, glucose et bicarbonate, et du chlorure en une quantité permettant de maintenir l'équilibre électrochimique.

14. Kit de composants contenant (i) un liquide de dialyse selon la revendication 11 ou 12
- ou un concentré selon la revendication 13 et (ii) un liquide de substitution, contenant du calcium, ou un concentré pour un tel liquide de substitution.

15. Procédé de dialyse extracorporelle ou de diafiltration de sang, comprenant
- la dialyse ou la diafiltration du sang contre un liquide de dialyse contenant du citrate, la concentration de citrate étant dans la plage de plus de 0,7 mmol/l et 2 mmol/l ou moins, ledit liquide de dialyse étant essentiellement exempt de calcium et essentiellement exempt de magnésium, sans ajout de citrate ou d'un autre anticoagulant au sang, en amont du dialyseur ; et
- le mélange du sang dialysé avec un liquide de substitution contenant du calcium et, de préférence, du magnésium,
où le terme essentiellement exempt désigne une concentration inférieure à 0,1 mmol/l.
